# EUROPEAN PATENT APPLICATION

(11) **EP 2 211 181 A1**
(43) Date of publication of application: **28.07.2010**
(21) Application number: 10160086.4
(22) Date of filing: 23.10.2003
(51) Int. Cl.: G01N 33/68

(54) **Diagnostic method for transmissible spongiform encephalopathies**

(30) Priority: 30.10.2002 GB 0225245; 19.03.2003 GB 0306290
(62) Divisional of application: 03758352.3
(71) Applicant: PROTEOME SCIENCES PLC, Cobham, Surrey KT11 3EP (GB)
(72) Inventor: Sanchez, Jean-Charles, 1233 Bernex (CH); Hochstrasser, Denis, CH-1245, Geneva (CH); Guillaume, Elisabeth, 1220, Divonne Les Bains (FR)
(74) Representative: Cole, Paul Gilbert

(57) **Abstract**

A method of diagnosis of a transmissible spongiform encephalopathy (TSE) or the possibility thereof in a subject suspected of suffering from the TSE, which comprises determining a test amount of a polypeptide in a sample of body fluid taken from the subject, wherein the polypeptide is differentially contained in the body fluid of TSE-infcctcd subjects and non-TSE-infcctcd subjects, and is Cystatin C; and determining whether the test amount is consistent with a diagnosis of TSE.

SEQUENCE LISTING

<110>  Proteome Sciences Plc

<120>  DIAGNOSTIC METHOD FOR TRANSMISSIBLE SPONGIFORM ENCEPHALOPATHIES

<130>  ELEC,023

<140>  GB 0306290.8
<141>  2003-03-19

<150>  GB 0225245.0
<151>  2002-10-30

<160>  2

<170>  PatentIn version 3.1

<210>  1
<211>  146
<212>  PRT
<213>  Homo sapiens

<400>  1

Met Ala Gly Pro Leu Arg Ala Pro Leu Leu Leu Leu Ala Ile Leu Ala
1               5                   10                  15

Val Ala Leu Ala Val Ser Pro Ala Ala Gly Ser Ser Pro Gly Lys Pro
            20                  25                  30

Pro Arg Leu Val Gly Gly Pro Met Asp Ala Ser Val Glu Glu Glu Gly
        35                  40                  45

Val Arg Arg Ala Leu Asp Phe Ala Val Gly Glu Tyr Asn Lys Ala Ser
    50                  55                  60

Asn Asp Met Tyr His Ser Arg Ala Leu Gln Val Val Arg Ala Arg Lys
65                  70                  75                  80

Gln Ile Val Ala Gly Val Asn Tyr Phe Leu Asp Val Glu Leu Gly Arg
                85                  90                  95

Thr Thr Cys Thr Lys Thr Gln Pro Asn Leu Asp Asn Cys Pro Phe His
            100                 105                 110

Asp Gln Pro His Leu Lys Arg Lys Ala Phe Cys Ser Phe Gln Ile Tyr
        115                 120                 125

Ala Val Pro Trp Gln Gly Thr Met Thr Leu Ser Lys Ser Thr Cys Gln
    130                 135                 140

Asp Ala
145

<210>  2

<211>  120

<212>  PRT

<213>  Homo sapiens

<400>  2

Ser Ser Pro Gly Lys Pro Pro Arg Leu Val Gly Gly Pro Met Asp Ala
1               5                   10                  15

Ser Val Glu Glu Glu Gly Val Arg Arg Ala Leu Asp Phe Ala Val Gly
            20                  25                  30

Glu Tyr Asn Lys Ala Ser Asn Asp Met Tyr His Ser Arg Ala Leu Gln
        35                  40                  45

Val Val Arg Ala Arg Lys Gln Ile Val Ala Gly Val Asn Tyr Phe Leu
    50                  55                  60

Asp Val Glu Leu Gly Arg Thr Thr Cys Thr Lys Thr Gln Pro Asn Leu
65                  70                  75                  80

Asp Asn Cys Pro Phe His Asp Gln Pro His Leu Lys Arg Lys Ala Phe
                85                  90                  95

Cys Ser Phe Gln Ile Tyr Ala Val Pro Trp Gln Gly Thr Met Thr Leu
            100                 105                 110

Ser Lys Ser Thr Cys Gln Asp Ala
        115                 120

## Description

### BACKGROUND OF THE INVENTION

### Field of the invention

This invention relates to a method for obtaining information that may have utility in providing an indication of the presence of a transmissible spongiform encephalopathy (TSE) or the possibility or progress thereof.

### Description of the related art

Transmissible spongiform encephalopathies (TSEs) are neurodegenerative diseases of the central nervous system. They can be transmitted, inherited or occur sporadically and are observed in animals, e.g. as bovine spongiform encephalopathy (BSE) in cattle or scrapie in sheep, as well as in humans as Creutzfeldt-Jakob disease (CJD), Gerstman Sträussler Scheinker syndrome, Fatal Familial Insomnia or Kuru. They have a long incubation period, leading to ataxia, dementia, psychiatric disturbances and death. Neuropathological changes include vacuolar degeneration of brain tissue, astrogliosis and amyloid plaque formation. The diseases are difficult to diagnose pre-mortem.

The cerebrospinal fluid (CSF) of CJD patients displays two additional polypeptides (known as 14-3-3 polypeptides) by two-dimensional polyacrylamide gel electrophoresis [Harrington, M.G. New England Journal of Medicine 315, 279 (1986), Hsich, G., Kenney, K., Gibbs, C.J., Lee, K.H. & Harrington, M. B. New England Journal of Medicine 335, 924 (1996).] The function of these 14-3-3 polypeptides remains unclear in TSE. They can be used in a pre-mortem test for CJD diagnostic evaluation, but have low specificity.

Monoclonal antibodies to the abnormal form of prion protein (which is associated with CJD) are available and can be used in an enzyme-linked immunoassay, as described in PCT Specifications WO 98/23962 and 98/32710 and Schmerr, M.J., the Beckman Coulter Pace Setter Newsletter 3(2), 1-4 (June 1999), but these procedures have not yet been fully developed.

WO 01/67108 relates to a diagnostic assay for TSEs in which the concentration of heart or brain fatty acid binding protein (H-FABP or B-FABP) is determined in a sample of body fluid.

WO 03/023406 (published 20 March 2003) describes a method for aiding TSE diagnosis, which comprises determining a test amount of a polypeptide marker, which is differentially present in samples of a TSE subject and a subject who does not have TSE. The marker may be determined in body fluids using mass spectrometry, and preferably laser desorption mass spectrometry.

US-A-6225047 describes the use of retentate chromatography to generate difference maps, and in particular a method of identifying analytes that are differentially present between two samples. One specific method described therein is laser desorption mass spectrometry.

WO 01/25791 describes a method for aiding a prostate cancer diagnosis, which comprises determining a test amount of a polypeptide marker, which is differentially present in samples of a prostate cancer patient and a subject who does not have prostate cancer. The marker may be determined using mass spectrometry, and preferably laser desorption mass spectrometry.

Development of new non-invasive TSE markers for body fluids or other body tissues (in particular, CJD and BSE markers in blood) and new methods of determining the markers would help clinicians to establish early diagnosis. This problem has now been solved by the present invention.

### SUMMARY OF THE INVENTION

The present invention provides a method of diagnosis of a transmissible spongiform encephalopathy (TSE) or the possibility thereof in a subject suspected of suffering from the TSE, which comprises subjecting a sample of body fluid taken from the subject to mass spectrometry, thereby to determine a test amount of a polypeptide in the sample, wherein the polypeptide is differentially contained in the body fluid of TSE-infected subjects and non-TSE-infected subjects, and has a molecular weight in the range of from 1000 to 100000, but excluding the range of from 3500 to 30000; and determining whether the test amount is consistent with a diagnosis of TSE.

The invention also provides use of a polypeptide which is differentially contained in a body fluid of TSE-infected subjects and non-infected subjects, the polypeptide having a molecular weight in the range of from 1000 to 100000, but excluding the range of from 3500 to 30000, and being determinable by mass spectrometry, for diagnostic, prognostic and therapeutic applications.

In embodiments of the invention, the molecular weight may, for example, be from 1000 to less than 3500, or from above 30000 to 100000, or selected from specific values in the range of from 3500 to 30000, as described below.

The invention further relates to the use of a marker of molecular weight about 13350 in a method of diagnosis of a transmissible spongiform encephalopathy (TSE) or the possibility thereof in a subject suspected of suffering from TSE. That marker is believed to be cystatin C (Swiss-Prot Accession NO: P01034, active protein of 120 AA, theoretical pI of 8.75) also called Neuroendocrine basic polypeptide, Gamma Trace or Post gamma globulin. This secreted active inhibitor of cysteine proteinases belongs to a super-family of proteins that includes 3 groups on the basis of similar sequence and structural properties. This protein is highly expressed in the epididymis, vas deferens, brain, thymus, and ovary and at a lower level in the submandibular gland. Cystatin C has been confirmed by immunoblotting to be differentially expressed in the CSF of CJD affected patients. It is derived from a precursor having the sequence (SEQ ID No: 1):
magplrapll llailavala vspaagsspg kpprlvggpm dasveeegvr raldfavgey 60
nkasndmyhs ralqwrark qivagvnyfl dvelgrttct ktqpnldncp fhdqphlkrk 120
afcsfqiyav pwqgtmtlsk stcqda 146
and has the sequence (SEQ ID No: 2) set out below:
sspgkpprlv ggpmdasvee egvrraldfa vgeynkasnd myhsralqvv rarkqivagv 60
nyfldvelgr ttctktqpnl dncpfhdqph lkrkafcsfq iyavpwqgtm tlskstcqda 120

A mutant form of cystatin C (Leu to Glu substitution in 68) has been described as being implicated in a hereditary form of cerebral hemorrhage characterized by a thickening of the cerebral artery walls, with deposition of material with the characteristics of amyloid. There are also some genotypes (BB: Ala to Thr substitution) associated with increased risk of late onset of Alzheimer disease.

The invention therefore provides a method of diagnosis of a transmissible spongiform encephalopathy (TSE) or the possibility thereof in a subject suspected of suffering from the TSE, which comprises subjecting a sample of body fluid taken from the subject to mass spectrometry, thereby to determine a test amount of a polypeptide in the sample, wherein the polypeptide is differentially contained in the body fluid of TSE-infected subjects and non-TSE-infected subjects, and is cystatin C; and determining whether the test amount is consistent with a diagnosis of TSE. The body fluid is preferably cerebrospinal fluid but may be whole blood, plasma, serum, urine or a tissue in which prion proteins tend to accumulate e.g. the tonsil and other tissues of the lympho-reticular system such as the lymph nodes.

The invention further provides the use of a level of cystatin C measurable or detectable in a sample of body tissue by mass spectroscopy and differentially contained in the body tissue of TSE-infected subjects and non-TSE-infected subjects as a marker for providing an indication of a transmissible spongiform encephalopathy (TSE) or the possibility or progress thereof in a subject liable to suffer from the TSE.

In further experiments the inventors have identified and validated various haemoglobin isoforms as being the main protein able to discriminate between BSE+ and BSE- affected cattle using laser desorption/ionization mass spectrometry. In SELDI mass spectroscopy peaks or clusters at about 30,000, 15000 Da, 7500 Da may be indicative of haemoglobin, and these may correspond to a substantially intact haemoglobin molecule with a multiple electrical charge, or it may be a haemaglobin chain or a truncated version or fragment thereof having an immunological reaction to antibodies specific for bovine haemoglobin. The presence or absence of haemoglobin peaks or clusters in a SELDI spectrum therefore provides a means for ante-mortem diagnosis for BSE in cattle e.g. by tests carried out on plasma or other body fluids.

In a further aspect, therefore, the invention provides a method of diagnosis of a transmissible spongiform encephalopathy (TSE) or the possibility thereof in a bovine subject suspected of suffering from the TSE, which comprises subjecting a sample of body fluid taken from the subject to mass spectrometry, thereby to determine a test amount of a polypeptide in the sample, wherein the polypeptide is differentially contained in the body fluid of TSE-infected bovine subjects and non-TSE-infected subjects, and is a haemoglobin, a haemoglobin chain or a truncated chain or a fragment thereof having an immunological reaction to antibodies specific for bovine haemoglobin; and determining whether the test amount is consistent with a diagnosis of TSE.

As used herein the expression "bovine" shall include cattle generally, sheep (scrapie) and also deer and elk (chronic wasting disease).

The invention further provides the use of a level of a haemoglobin, a haemoglobin chain or a truncated chain or a fragment thereof thereof having an immunological reaction to antibodies specific for bovine haemoglobin, said level being measurable or detectable in a sample of body tissue by mass spectroscopy and said a haemoglobin, a haemoglobin chain or a truncated chain or a fragment thereof which exhibits an immunological reaction to an antibody to bovine haemoglobin being differentially contained in the body tissue of bovine TSE-infected subjects and non-bovine non-TSE-infected subjects as a marker for providing an indication of a transmissible spongiform encephalopathy (TSE) or the possibility or progress thereof in a bovine subject liable to suffer from the TSE.

The above test will be of value when applied to an animal or herd of animals either on a single occasion or at intervals, and animals that have been found not to be suffering from an actual or latent transmissible spongiform encephalopathy will self-evidently be of enhanced value. Such animals and the method of testing them are also within the scope of the invention.

The invention further provides assay devices or kits for use in the diagnosis of a TSE comprising a solid substrate having attached thereto an antibody that is specific for any of the following:
(i) a polypeptide that is differentially contained in the body fluid of TSE-infected subjects and non-TSE-infected subjects, and has a molecular weight in the range of from 1000 to 100000;
(ii) a polypeptide that is differentially contained in the body fluid of TSE-infected subjects and non-TSE-infected subjects, and is selected from those having a respective molecular weight of about 1010, 1100, 1125, 1365, 3645, 4030, 3890, 5820, 7520, 7630, 7980, 9950, 10250, 11600, 11800, 15000, 15200, 15400, 15600, 15900,30000,31000 and 31800 Da.;
(iii) cystatin C;
(iv) a haemoglobin, a haemoglobin chain or a truncated chain or a fragment thereof which exhibits an immunological reaction to an antibody to bovine haemoglobin and is differentially contained in the body tissue of bovine TSE-infected subjects and non-bovine non-TSE-infected subjects. The above devices or kits may further comprise necessary preparative reagents, washing reagents, detection reagents and signal producing reagents.

The invention also further provides an assay device for use in the diagnosis of TSE which comprises any of
a plate having a location containing a material which recognizes, binds to or has affinity for a polypeptide which is differentially contained in a body fluid of TSE-infected subjects and non-infected subjects, the polypeptide having a molecular weight in the range of from 1000 to 100000 and being determinable by mass spectrometry;
a plate having a location containing an antibody that is specific for Cystatin C;
a plate having a location containing an antibody that is specific for Cystatin C and useful in the diagnosis of variant CJD;
a plate having a location containing an antibody that is specific for Cystatin C and useful in the diagnosis of sporadic CJD. The above devices may also be provided in association with necessary preparative reagents, washing reagents, detection reagents and signal producing reagents.

The invention further provides a kit for use in diagnosis of TSE, comprising a probe or a protein chip array for receiving a sample of body fluid, and for placement in a mass spectrometer, thereby to determine a test amount of a polypeptide in the sample, wherein the polypeptide is differentially contained in the body fluid of TSE-infected subjects and non-TSE-infected subjects, and has a molecular weight in the range of from 1000 to 100000.

### BRIEF DESCRIPTION OF THE DRAWINGS

Figures 1A to 1F are spectral views of plasma from normal and BSE-infected samples using laser desorption/ionization mass spectrometry, highlighting protein peaks at about 1010, 1100, 1125, 1365, 3645, 4030, 3890, 5820, 7520, 7630, 7980, 9950, 10250, 11600, 11800, 15000, 15200, 15400, 15600, 15900, 30000, 31000 and 31800 Da in plasma samples;
Figure 2 shows human cystatin C immunodetection in CSF samples;
Fig. 3 shows bovine haemoglobin detection in plasma samples;
Fig. 4 shows a BSE plasma sample on a two-dimensional gel and on a two-dimensional PVDF membrane;
Fig 5 shows spectra of human and bovine haemoglobin using laser desorption/ionization mass spectrometry; and
Fig. 6A-6C show spectra from plasma from normal bovine and BSE-infected samples using laser desorption/ionization mass spectrometry.

### DESCRIPTION OF PREFERRED EMBODIMENTS

The invention provides a method of diagnosis of a transmissible spongiform encephalopathy (TSE) or the possibility thereof in a subject suspected of suffering from the TSE. A sample of body fluid taken from the subject is subjected to mass spectrometry, to determine the presence or absence in the sample of a polypeptide marker, which is differentially contained in the body fluid of TSE-infected subjects and non-infected subjects. The polypeptide marker has a molecular weight in the range of from 1000 to 100000, preferably from 1000 to 35000, and the presence, absence, under-expression or over-expression of the marker is indicative of TSE.

The method is applicable to all types of TSE, and to any human or animal suffering or suspected of suffering therefrom. The method is especially applicable to the diagnosis of CJD, especially new variant CJD, in human patients, and to BSE in ruminant animals such as cattle, and to BSE-like diseases in other animals, such as scrapie in sheep.

The term polypeptide includes proteins and protein fragments, as well as peptides modified by the addition of non-peptide residues, e.g. carbohydrates, phosphates, sulfates or any other post-translational modification.

The sample may be adsorbed on a probe under conditions which allow binding between the polypeptide and adsorbent material on the probe or the protein chip array. The adsorbent material preferably comprises a metal chelating group complexed with a metal ion, and a preferred metal is copper. Prior to detecting the polypeptide, unbound or weakly bound materials on the probe or protein chip array may be removed with a washing solution, thereby enriching the polypeptide in the sample. The sample is preferably adsorbed on a probe or protein chip array having an immobilised metal affinity capture (IMAC) surface capable of binding the polypeptide. The sample may be also adsorbed on a probe having hydrophobic, strong anionic or weak cationic exchange surfaces under conditions which allow binding of the polypeptides. The probe may consist of a strip having several adsorbent wells, and be inserted into the spectrometer, then movable therein so that each well is in turn struck by the ionizing means (e.g. laser) to give a spectrometer reading. The polypeptide is preferably determined by surface-enhanced laser desorption/ionisation (SELDI) and time of flight mass spectrometry (TOF-MS).

In principle, any body fluid or tissue can be used to provide a sample for diagnosis, but preferably the body fluid is cerebrospinal fluid (CSF), plasma, serum, blood, urine, saliva or tears.

In one embodiment of the invention, the TSE is bovine spongiform encephalopathy (BSE). In this case, the polypeptide preferably has a molecular weight of about 1010, 1100, 1125, 1365, 3645, 4030, 3890, 5820, 7520, 7630, 7980, 9950, 10250, 11600, 11800, 15000, 15200, 15400, 15600, 15900, 30000, 31000 and 31800 Da and the presence, absence, over-expression or under-expression of the polypeptide is indicative of BSE. It will be appreciated that the invention embraces making a measurement at any one or more of the foregoing molecular weight values, in any combination thereof, for the purpose of making a diagnosis.

In another embodiment of the invention, the TSE is CJD.

In a further embodiment of the invention, the TSE is scrapie

Measurement of the molecular weight of the polypeptide or polypeptides is effected in the mass spectrometer. All molecular weights herein are measured in Da. The molecular weights quoted above can be measured with an accuracy of better than 1%, generally 0.5 to 1%, and preferably to within about 0.1%. The term "about" in connection with molecular weights in this specification therefore means within a variation of about 1%, preferably 0.5%, and more preferably within about 0.1%, above or below the quoted value.

The invention also relates to the use of a polypeptide which is differentially contained in a body fluid of TSE-infected subjects and non-infected subjects, the polypeptide having a molecular weight in the range of from 1000 to 100000, but excluding the range of from 3500 to 30000 and being determinable by mass spectrometry, for diagnostic, prognostic and therapeutic applications. This may involve the preparation and/or use of a material, which recognizes, binds to or has some affinity to the above-mentioned polypeptide. Examples of such materials are antibodies and antibody chips. The term "antibody" as used herein includes polyclonal antiserum, monoclonal antibodies, fragments of antibodies such as Fab, and genetically engineered antibodies. The antibodies may be chimeric or of a single species. The above reference to "prognostic" applications includes making a determination of the likely course of a TSE by, for example, measuring the amount of the above-mentioned polypeptide in a sample of body fluid. The above reference to "therapeutic" applications includes, for example, preparing materials which recognize, bind to or have affinity to the above-mentioned polypeptides, and using such materials in therapy. The materials may in this case be modified, for example by combining an antibody with a drug, thereby to target the drug to a specific region of the animal to be treated.

The methodology of this invention can be applied to the diagnosis of any TSE. Body fluid samples are prepared from infected and non-infected subjects. The samples are applied to a probe or array having a surface treated with a variety of adsorbent media, for differential retention of peptides in the sample, optionally using washing liquids to remove unbound or weakly bound materials. If appropriate, energy-absorbing material can also be applied. The probe or array is then inserted into a mass spectrometer, and readings are taken for the various sample/adsorbent combinations using a variety of spectrometer settings. Comparison of the infected and non-infected samples under a given set of conditions reveals one or more polypeptides, which are differentially expressed in the infected and non-infected samples. The presence or absence of these polypeptides can then be used in the testing of a fluid sample from a subject under the same conditions (adsorbent, spectrometer settings etc.) to determine whether or not the subject is infected.

References herein to "presence or absence" of a polypeptide should be understood to mean simply that there is a significant difference in the amount of a polypeptide which is detected in the infected and non-infected sample. Thus, the "absence" of a polypeptide in a test sample may include the possibility that the polypeptide is actually present, but in a significantly lower amount than in a comparative test sample. According to the invention, a diagnosis can be made on the basis of the presence or absence of a polypeptide, and this includes the presence of a polypeptide in a significantly lower or significantly higher amount with reference to a comparative test sample.

The following Examples illustrate the invention.

### EXAMPLE 1

### Polypeptides in body fluids (cerebrospinal fluid, plasma and others) of BSE affected cattle

The objective of the present study was to detect specific polypeptides in body fluids (cerebrospinal fluid, plasma and others) of BSE affected cattle. Samples were analysed by the Surface Enhanced Laser Desorption Ionization (SELDI) Mass Spectroscopy (MS) technology. This technology encompasses micro-scale affinity capture of proteins by using different types of retentate chromatography and then analysis by time of flight mass spectrometry. Different maps are thus generated each corresponding to a typical protein profiling of given samples that were analysed with a Ciphergen Biosystem PBS II mass spectrometer (Freemont, CA, USA). Differential expressed peaks were identified when comparing spectra generated in a group of plasma samples from BSE-affected cattle with a group of healthy cattle using protein chip arrays.

The SELDI analysis was performed using 2µl of crude bovine plasma samples in order to detect specific polypeptides with metal affinity. An immobilized copper affinity array (IMAC-Cu⁺⁺) was employed in this approach to capture proteins with affinity for copper to select for a specific subset of proteins from the samples. It will be appreciated that other protein chip arrays and immobilized metal chip arrays may be substituted for the IMAC-Cu⁺⁺ affinity array. Captured proteins were directly detected using the PBSII Protein Chip Array reader (Ciphergen Biosystems, Freemont, CA, USA).

The following protocol was used for the processing and analysis of ProteinChip arrays using Chromatographic TED-Cu(II) adsorbent array. TED is a (tris(carboxymethyl)ethylenediamine-Cu) adsorbent coated on a silicon oxide-coated stainless steel substrate.
- The surface was first loaded with 10 µl of 100 mM copper sulfate to each spot and incubated for 15 minutes in a wet chamber.
- The chip was thereafter washed by two quick rinses with deionized water for about 10 seconds to remove the excess unbound copper.
- Before loading the samples, the I-MAC 3 array was equilibrated once with 5 µl of PBS NaCl 0.5 M for 5 minutes.
- After removing the equilibration buffer, 3 µl of the same buffer were added before applying 2 µl of plasma. The chip was incubated for 20 minutes in a wet chamber.
- The samples were thereafter removed and the surface was washed three times with the equilibration buffer (5 minutes each).
- Two quick final rinses with water were performed.
- The surface was allowed to air dry, followed by the addition of 0.5 µl of saturated sinapinic acid (SPA, Ciphergen Biosystem) prepared in 50% acetonitrile, 0.5% trifluoroacetic acid.
- The chip was air dried again before analysis of the retained protein on each spot with laser desorption/ionization time-of-flight mass spectrometry.
- The protein chip array was inserted into the instrument and analysed once the appropriate detector sensitivity and laser energy have been established to automate the data collection.
- The obtained spectra were analysed with the Biomark Wizard software (Ciphergen Biosystems, Freemont, CA, USA) running on a Dell Dimension 4100 PC. It generates consistent peak sets across multiple spectra.

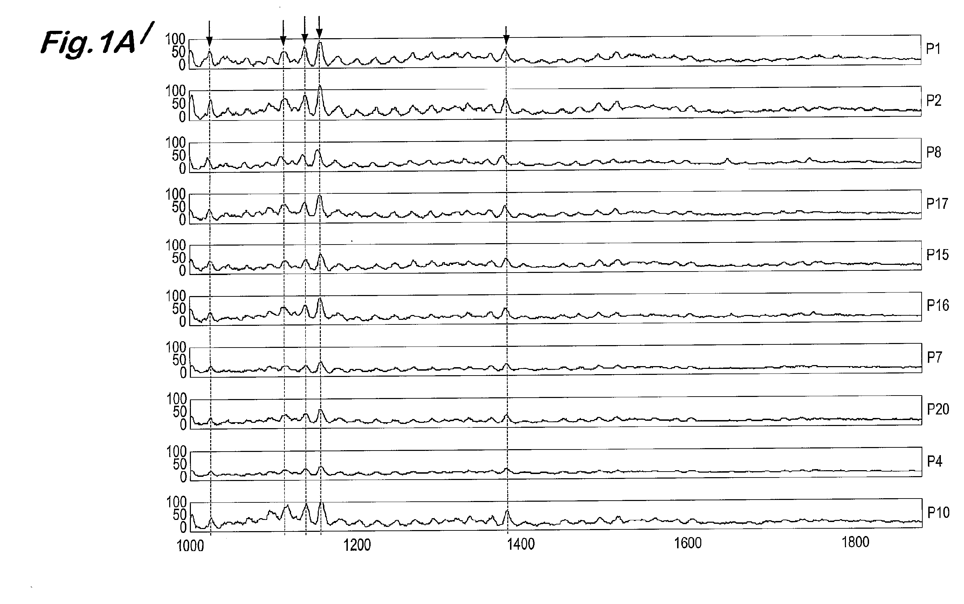

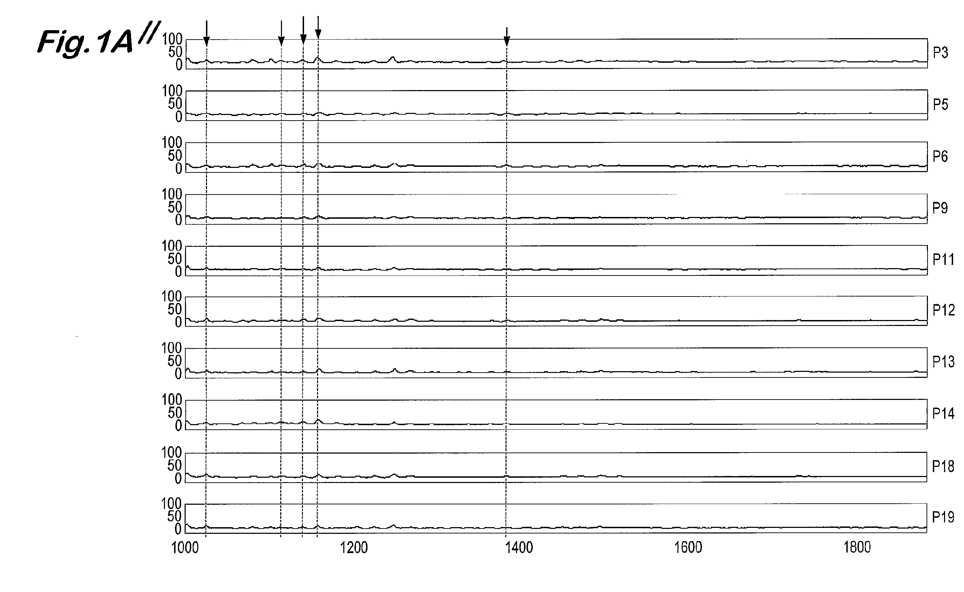

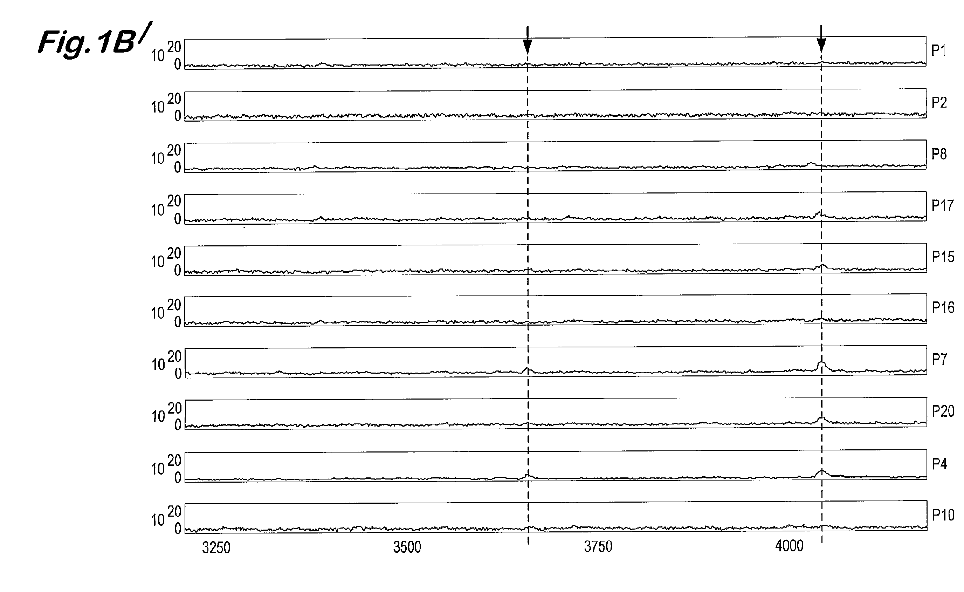

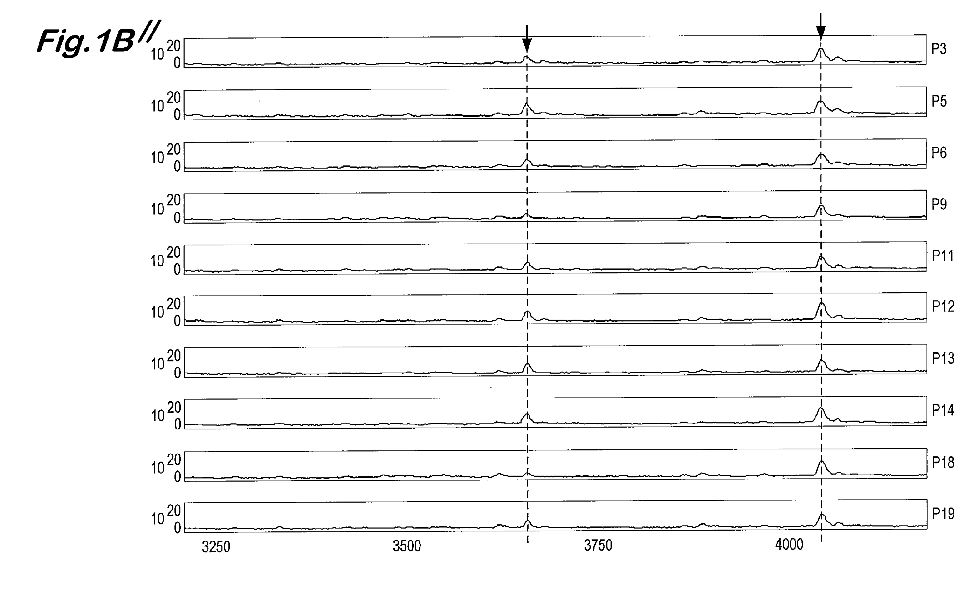

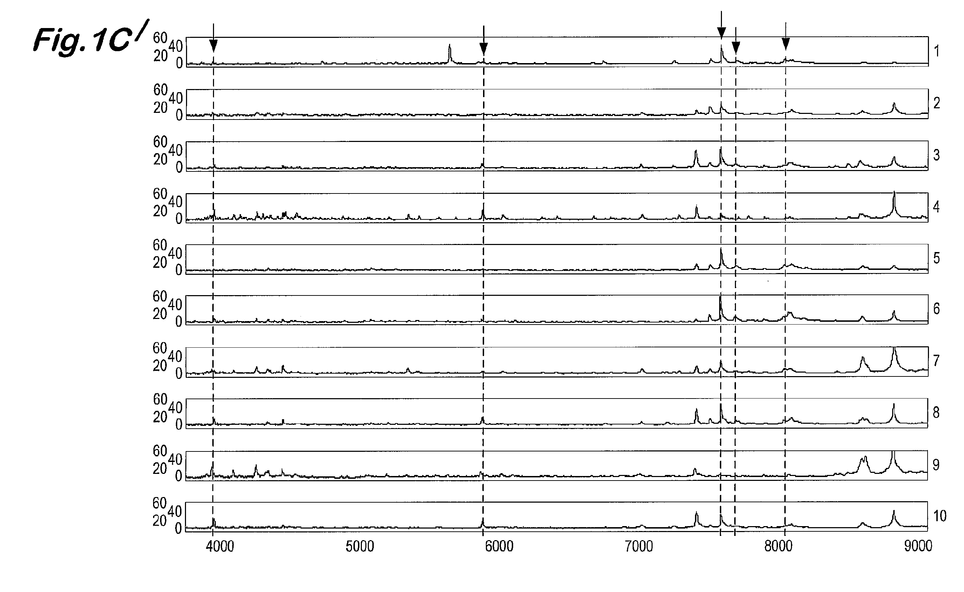

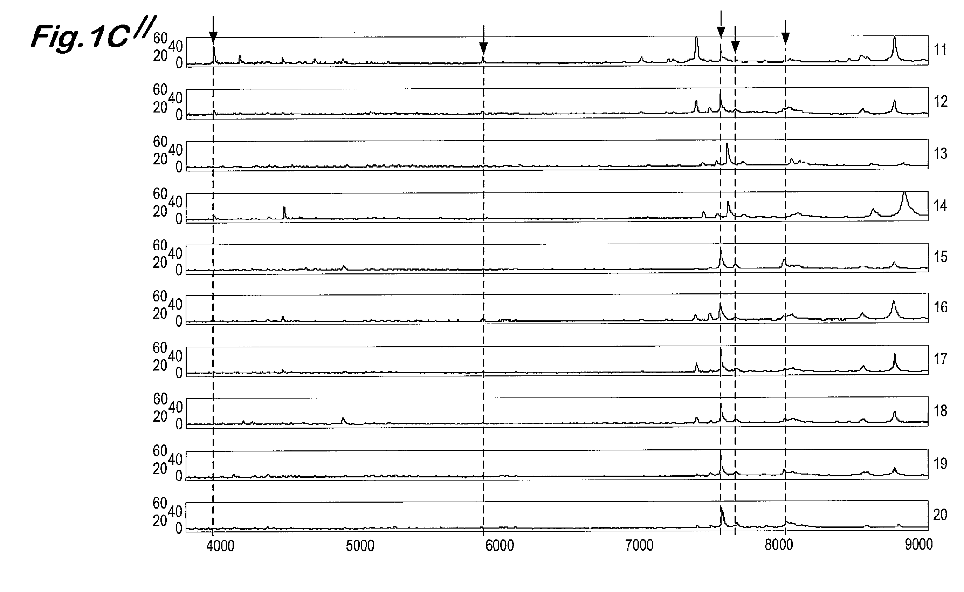

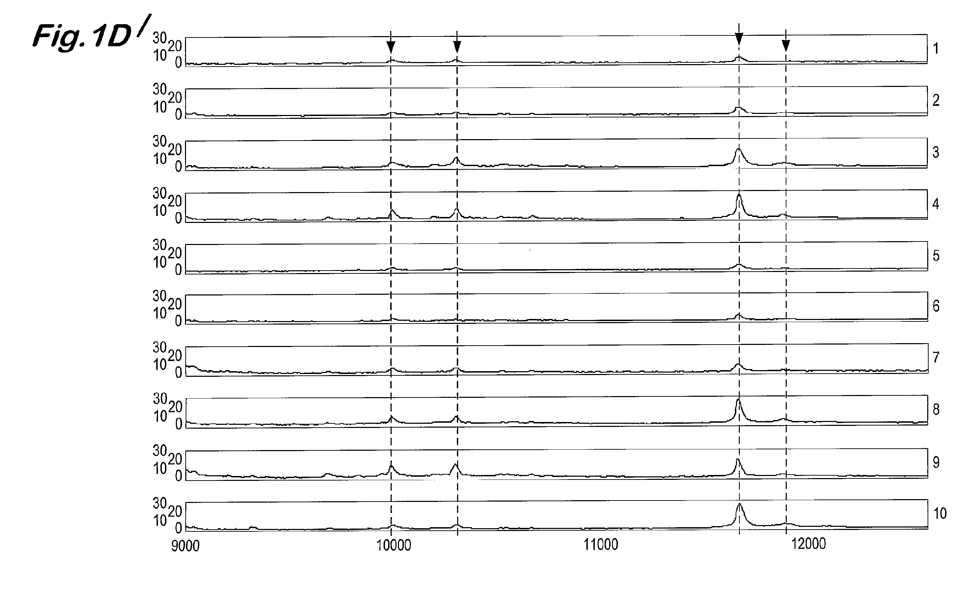

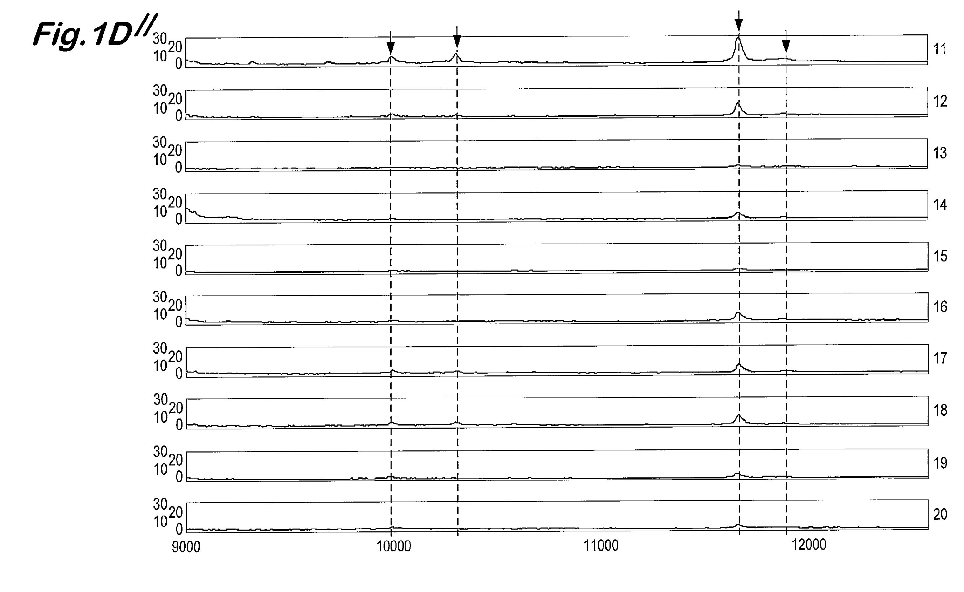

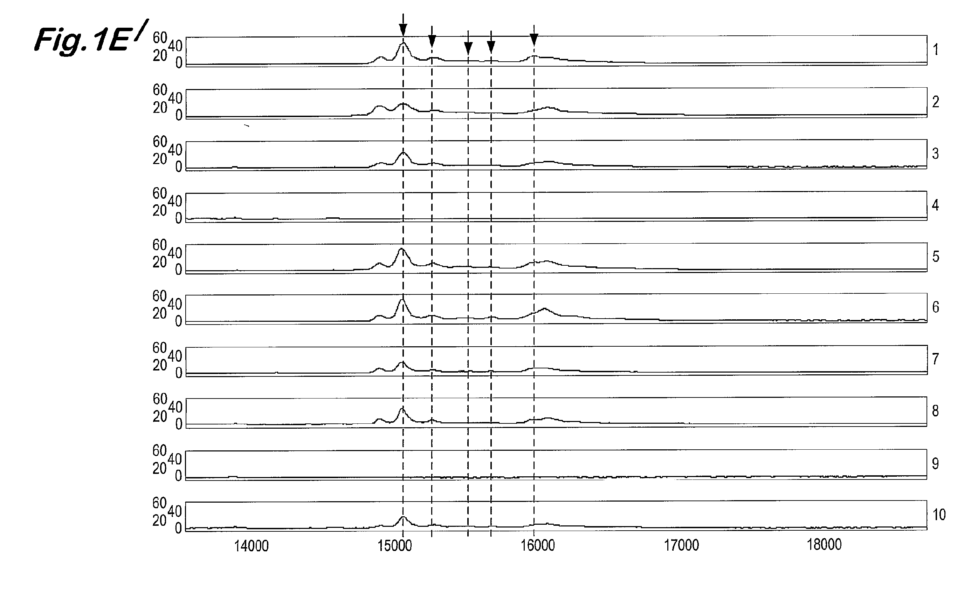

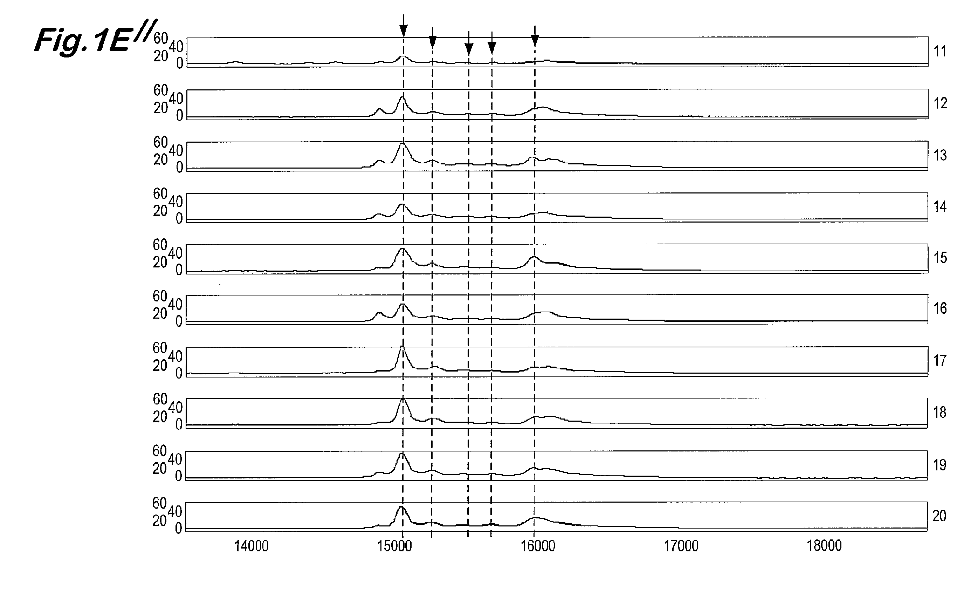

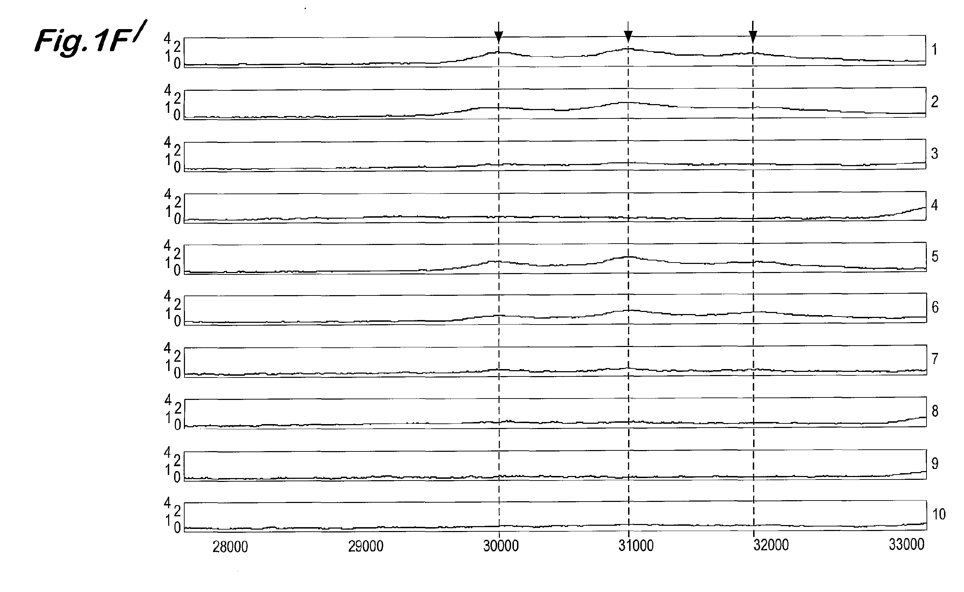

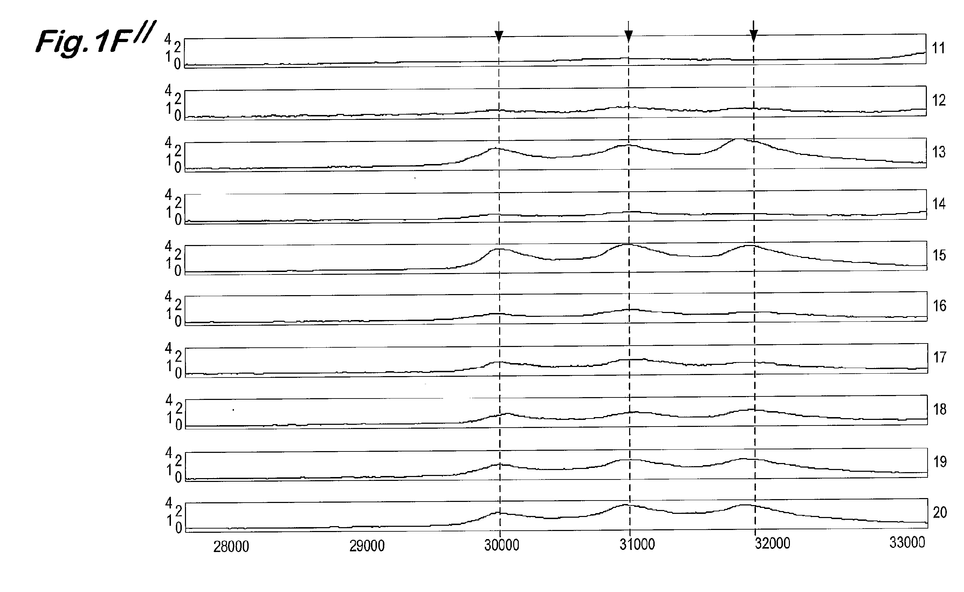

Figures 1A-1F show the results of a comparative study, which has been undertaken between plasma from BSE diagnosed cattle and normal plasma, using the IMAC3 protein chip array prepared as described above. In this study, we found that 23 peaks were significantly differentially expressed in plasma from BSE affected cattle. Their molecular weights are respectively about 1010, 1100, 1125, 1365, 3645, 4030, 3890, 5820, 7520, 7630, 7980, 9950, 10250, 11600, 11800, 15000, 15200, 15400, 15600, 15900, 30000, 31000 and 31800 Da (mass accuracy is around 0.1%). Figure 1 shows two spectral views, respectively of the normal and BSE samples, from 0 to 100,000 Da. More specifically, as indicated by the vertical arrows, Figure 1A shows the peaks at about 1010, 1100, 1125 and 1365. Figure 1B shows the peaks at about 3645 and 4030. Figure 1C shows the peaks at about 3890, 5820, 7520, 7630 and 7980. Figure 1D shows the peaks at about 9950, 10250, 11600 and 11800. Figure 1E shows the peaks at about 15000, 15200, 15400, 15600 and 15900. Figure 1F shows the peaks at about 30000, 31000 and 31800.

Spectra P1 to P20 (Figures 1A-1B) correspond to a batch of samples from UK, and spectra 1 to 20 (Figures 1C-1F) correspond to a batch of samples from US. The status of the cattle providing the samples is indicated below in Tables 1 and 2, where negative means not affected by BSE and positive means BSE affected cattle.

**Table 1**

| # | Type | Status |
|---|---|---|
| P1 | Plasma | Negative |
| P2 | Plasma | Negative |
| P3 | Plasma | Positive |
| P4 | Plasma | Negative |
| P5 | Plasma | Positive |
| P6 | Plasma | Positive |
| P7 | Plasma | Negative |
| P8 | Plasma | Negative |
| P9 | Plasma | Positive |
| P10 | Plasma | Negative |
| P11 | Plasma | Positive |
| P12 | Plasma | Positive |
| P13 | Plasma | Positive |
| P14 | Plasma | Positive |
| P15 | Plasma | Negative |
| P16 | Plasma | Negative |
| P17 | Plasma | Negative |
| P18 | Plasma | Positive |
| P19 | Plasma | Positive |
| P20 | Plasma | Negative |

**Table 2**

| # | Type | Status |
|---|---|---|
| 1 | Plasma | Positive |
| 2 | Plasma | Positive |
| 3 | Plasma | Positive |
| 4 | Plasma | Positive |
| 5 | Plasma | Positive |
| 6 | Plasma | Positive |
| 7 | Plasma | Positive |
| 8 | Plasma | Positive |
| 9 | Plasma | Positive |
| 10 | Plasma | Positive |
| 11 | Plasma | Positive |
| 12 | Plasma | Negative |
| 13 | Plasma | Negative |
| 14 | Plasma | Negative |
| 15 | Plasma | Negative |
| 16 | Plasma | Negative |
| 17 | Plasma | Negative |
| 18 | Plasma | Negative |
| 19 | Plasma | Negative |
| 20 | Plasma | Negative |

These data demonstrate that the peaks of about 1010, 1100, 1125, 1365, 3645, 4030, 3890, 5820, 7520, 7630, 7980, 9950, 10250, 11600, 11800, 15000, 15200, 15400, 15600, 15900, 30000, 31000 and 31800 Da can be used to diagnose BSE in plasma samples. Any one of the above peaks, or more than one of them in any combination, can be used in this way to diagnose BSE.

### EXAMPLE 2

### Identification and up-regulation of cystatin C in CSFs of CJD affected patients

CSF samples (100µl) obtained from the CJD surveillance unit (sporadic or definite variant of CJD, as well as Not Case) were investigated using SELDI protein Chip Array technology. WCX2 (a weak cation exchange array with carbohydrate functionality), SAX2 (a strong anion exchange array with quaternary amine functionality) and IMAC3 (an immobilized metal affinity capture array with nitrotriacetic acid) surfaces were used in order to investigate differential specific binding of the proteins in the samples obtained. Various comparisons were performed between firstly samples from definite and sporadic cases (CJD samples) versus corresponding controls (Not Case) in order to discriminate between samples from patients with CJD symptoms and samples from patients with similar symptoms which were not CJD (Not Case = Control). The whole groups of samples from CJD-diagnosed patients compared with controls from the institute and the inventors' 14.3.3 samples were analysed without any distinction in the diagnosed sub-population. Comparison of these samples did not allow sporadic and/or variant CJD samples to be distinguished from their corresponding controls (Not Case).

The inventors therefore confirmed the presence of a dementia marker of molecular weight about 13365-13370 (± 0.5%), which in addition to metal affinity also demonstrates cationic properties.

The 13365-13370 marker was purified by fractionating the Not Case CSF samples using anionic exchange chromatography. Spin Sax columns designed for such fractionation separate proteins according to their net charge and were used to elute proteins stepwise with decreasing pI using buffers with increasing salt concentration and decreasing pI. The protein profiles obtained using both IMAC3 and SAX chips were similar. Each fraction was loaded on a SDS PAGE for further identification of the 13350 mw peak. A preparative colloidal blue-stained Tris-Tricine 1-DE gel was run and stained with silver nitrate to permit band excision and digestion followed by mass spectrometry identification. Identification of the squared bands excised from the gel followed the MALDI-TOF, MALDI-TOF-TOF (Applied Biosystems: provides partia sequence determination using collision-induced dissociation fragment analysis) or nanoLC Q-tof approaches. The 13350 peak was identified as cystatin C.

Matrix-assisted laser desorption/ionisation-time of flight mass spectrometry (MALDI-TOF MS) is a relatively novel technique in which a co-precipitate of an UV-light absorbing matrix and a biomolecule is irradiated by a nanosecond laser pulse. Most of the laser energy is absorbed by the matrix, which prevents unwanted fragmentation of the biomolecule. The ionized biomolecules are accelerated in an electric field and enter the flight tube. During the flight in this tube, different molecules are separated according to their mass to charge ratio and reach the detector at different times. In this way each molecule yields a distinct signal. The method is used for detection and characterization of biomolecules, such as proteins, peptides, oligosaccharides and oligonucleotides, with molecular masses between 400 and 350,000 Da.

In order to validate the identification and the up-regulation of cystatin C in CSF's of CJD affected patients, the inventors performed Western blot experiments using an antibody specific for human cystatin C on 8 demented CSF samples versus 8 CSF of CJD affected patients (3 variant and 5 sporadic CJD).

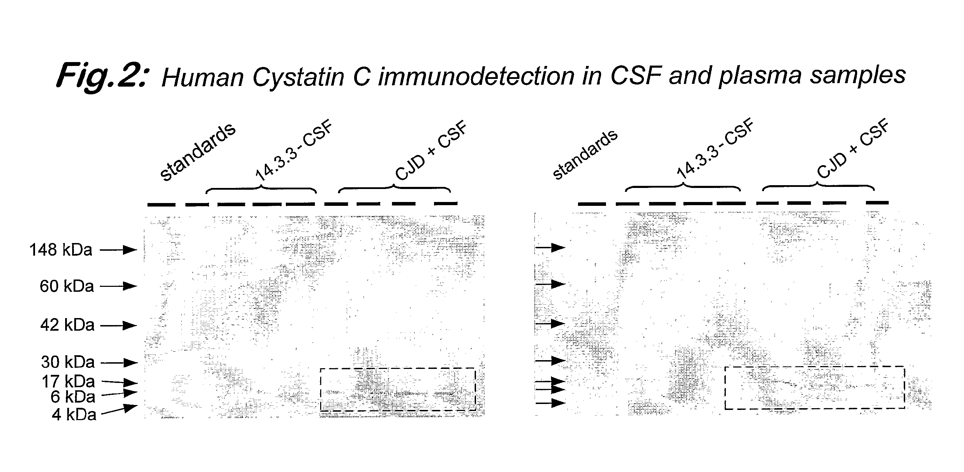

Figure 2 shows the specific increased signal that the inventors obtained in the 8 CJD affected patients tested in comparison to controls, showing that Cystatin provides a cerebrospinal fluid marker of CJD.

### EXAMPLE 3

### Plasma of BSE affected cattle: SELDI analysis.

Previous comparative studies using SELDI analysis of BSE+ and BSE- plasma samples allowed the inventors to highlight several protein clusters as being differentially expressed. One such cluster with a mass centered around 15000Da was selected for further analysis, being differentially over-expressed in samples from BSE-affected cattle. Plasma of BSE-affected cattle was subjected to one-DE electrophoresis and the band migrating with a mass of 15000 Da as determined using standard mass marker proteins was excised, digested with trypsin and subjected to mass spectrometry by MAL:DI-TOF. By this method the 15000 Da protein species in the SELDI analysis were putatively identified as isoforms of bovine haemoglobin.

In order to investigate and validate the hemoglobin content of these samples, the inventors looked for antibodies that could cross react with bovine Hb. A goat polyclonal antibody anti-human Hb that reacts weakly with equine and bovine hemoglobin (J16, Biomeda), as well as a sheep polyclonal antibody raised against native Hb from erythrocytes expected to exhibit cross-reactivity with Hb from other species (4870-3980, Biotrend-Anawa) were tested. Experiments were carried out using as positive controls, native purified human and bovine Hb (4870-4056 and 4870-2002, Biotrend-Anawa).

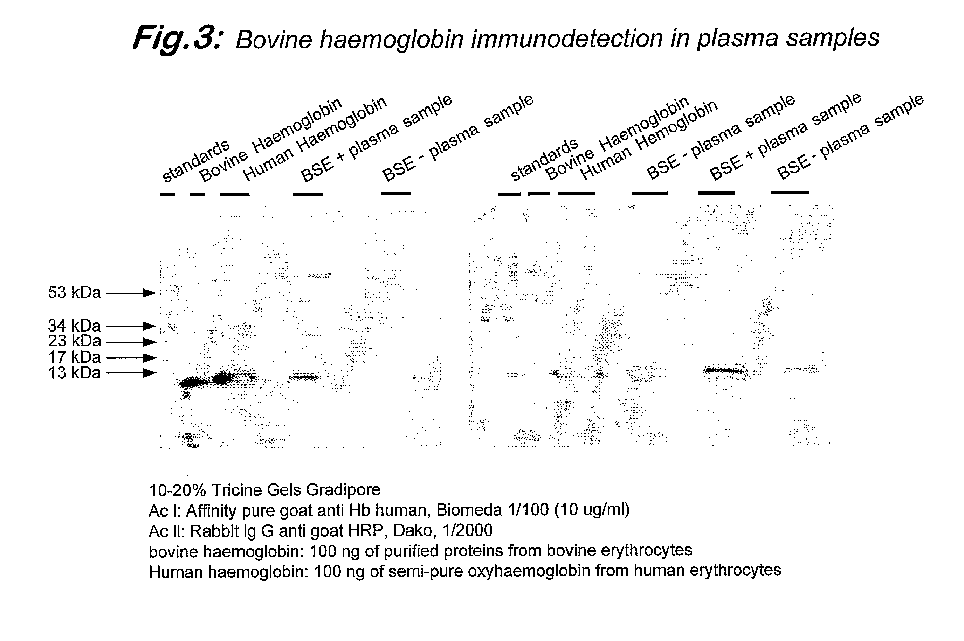

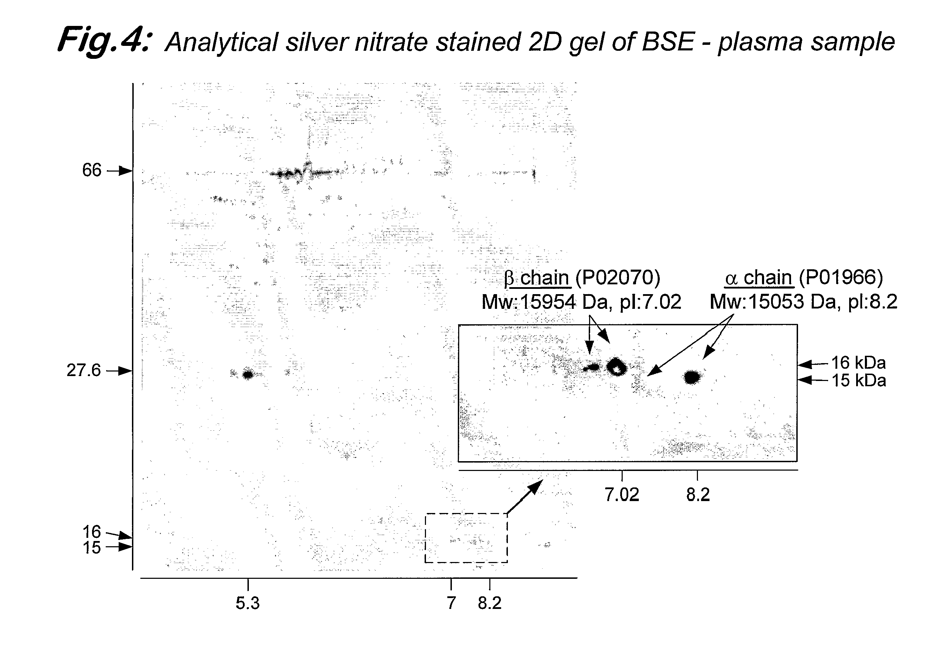

Figure 3 highlights the Western blot experiments performed with the goat polyclonal antibody from Biomeda. Besides the strong signal obtained with human and bovine hemoglobin, an increased level of a protein at the expected size in the 3 BSE+ tested plasma samples may indeed be observed. The Western blot experiments performed with the second antibody did not show any signal (data not shown). Silver stained analytical 2-DE gel (Figure 4) and an immunodetection experiment on 2-DE PVDF membranes of BSE- plasma sample were performed, in order to see if several spots could be thus detected. The enlarged portion of Figure 4 shows 4 spots in the expected area. Two spots seem to correspond to α chains (15053 Da, pI 8.19) and 2 others spots seem to correspond to β chains (15954 Da, pI 7.02). Knowing that the α chain has 4 putative modifications sites consisting of N-glycosylation, Protein Kinase C Phosphorylation, Casein Kinase 2-Phosphorylation and N-Myristoylation, as well as β chains having similar ones with an Amidation site instead of N-glycosylation one (Scan Prosite), these explain variations in their main molecular weight, as well as the numerous shoulders highlighted in SELDI spectra.

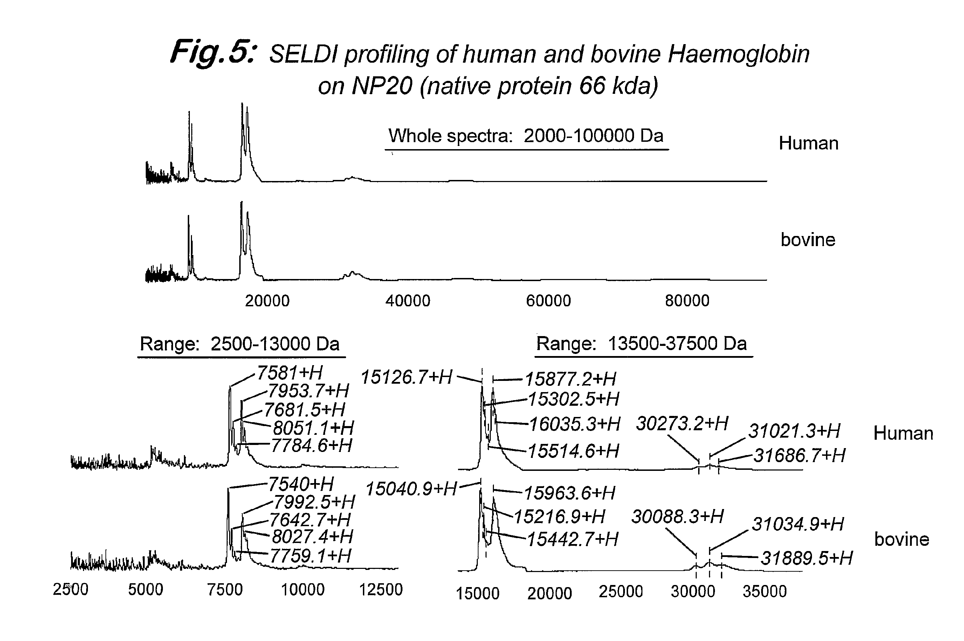

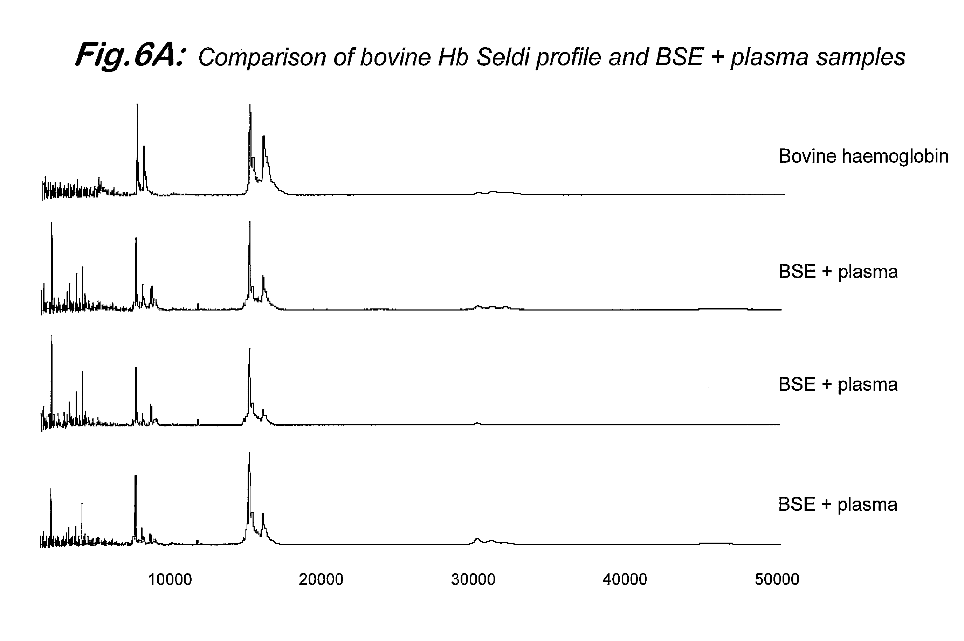

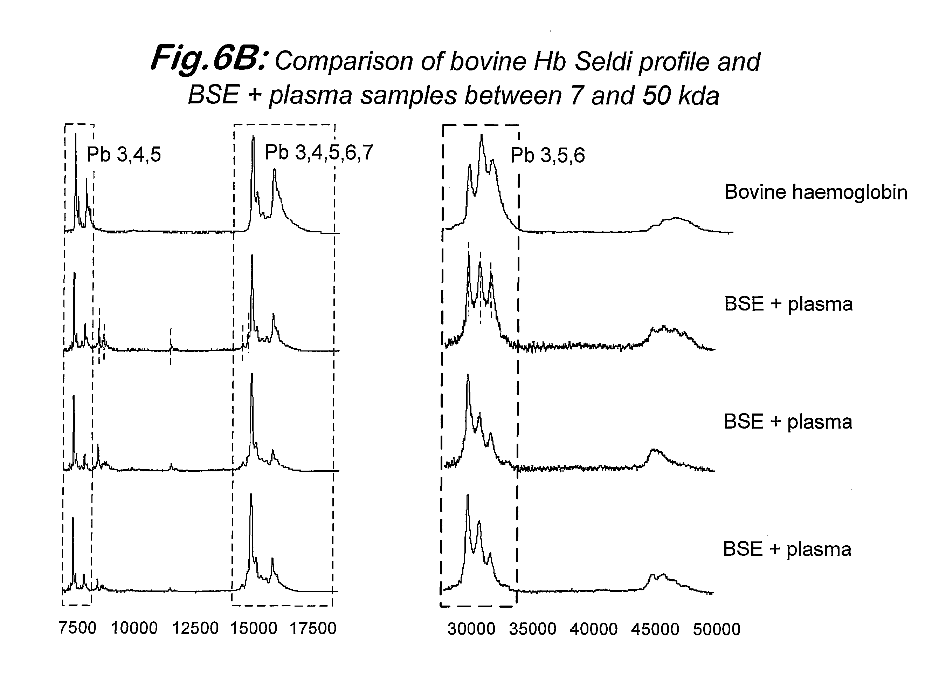

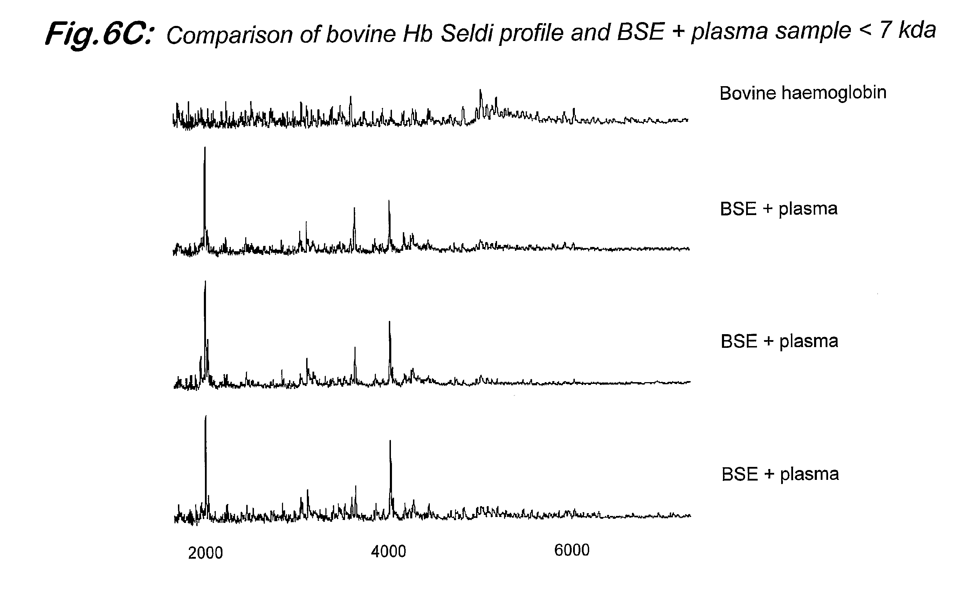

To further confirm the identity of the 15000 Da cluster as isoforms of bovine haemoglobin, bovine and human purified Hb were analysed on Normal phase with SELDI. Figure 5 shows the whole spectra obtained for both species, illustrating their similarity. Figures 6A and 6B point out within 4 representative spectra, how much the Hb profile looks like those of BSE+ plasma samples on I MAC, whilst Figure 6C highlights their difference under 7 kDa. These spectra led the inventors to conclude that 5 out of the 13 clusters highlighted in the earlier SELDI study of bovine plasma correspond to haemoglobin.

Each of the above cited publications is herein incorporated by reference to the extent to which it is relied on herein.

## Claims

1. A method of diagnosis of a transmissible spongiform encephalopathy (TSE) or the possibility thereof in a subject suspected of suffering from the TSE, which comprises determining a test amount of a polypeptide in a sample of body fluid taken from the subject, wherein the polypeptide is differentially contained in the body fluid of TSE-infected subjects and non-TSE-infected subjects, and is Cystatin C; and determining whether the test amount is consistent with a diagnosis of TSE.

2. A method of diagnosis of a transmissible spongiform encephalopathy (TSE) or the possibility thereof in a subject suspected of suffering from the TSE, which comprises subjecting a sample of body fluid taken from the subject to mass spectrometry, thereby to determine a test amount of a polypeptide in the sample, wherein the polypeptide is differentially contained in the body fluid of TSE-infected subjects and non-TSE-infected subjects, and is Cystatin C; and determining whether the test amount is consistent with a diagnosis of TSE.

3. The method of claim 1 or 2, wherein the body fluid is CSF.

4. A method of diagnosis of a transmissible spongiform encephalopathy (TSE) or the possibility thereof in a bovine subject suspected of suffering from the TSE, which comprises determining a test amount of a polypeptide in a sample of body fluid taken from the subject, wherein the polypeptide is differentially contained in the body fluid of TSE-infected bovine subjects and non-TSE-infected subjects, and is a haemoglobin, a haemoglobin chain or a truncated chain or a fragment thereof which exhibits an immunological reaction to an antibody to bovine haemoglobin; and determining whether the test amount is consistent with a diagnosis of TSE.

5. A method of diagnosis of a transmissible spongiform encephalopathy (TSE) or the possibility thereof in a bovine subject suspected of suffering from the TSE, which comprises subjecting a sample of body fluid taken from the subject to mass spectrometry, thereby to determine a test amount of a polypeptide in the sample, wherein the polypeptide is differentially contained in the body fluid of TSE-infected bovine subjects and non-TSE-infected subjects, and is a haemoglobin, a haemoglobin chain or a truncated chain or a fragment thereof which exhibits an immunological reaction to an antibody to bovine haemoglobin; and determining whether the test amount is consistent with a diagnosis of TSE.

6. Use of a level of cystatin C measurable or detectable in a sample of body tissue and differentially contained in the body tissue of TSE-infected subjects and non-TSE-infected subjects as a marker for providing an indication of a transmissible spongiform encephalopathy (TSE) or the possibility or progress thereof in a subject liable to suffer from the TSE.

7. The use of claim 6, in which the level is measurable or detectable by mass spectroscopy.

8. The use of claim 6 or 7, wherein the body tissue is from a human subject.

9. The use of claim 8, wherein the body tissue is cerebrospinal fluid.

10. Use of a level of a haemoglobin, a haemoglobin chain or a truncated chain or a fragment thereof which exhibits an immunological reaction to an antibody to bovine haemoglobin, said level being measurable or detectable in a sample of body tissue and said a haemoglobin, a haemoglobin chain or a truncated chain or a fragment thereof which exhibits an immunological reaction to an antibody to bovine haemoglobin being differentially contained in the body tissue of bovine TSE-infected subjects and non-bovine non-TSE-infected subjects as a marker for providing an indication of a transmissible spongiform encephalopathy (TSE) or the possibility or progress thereof in a bovine subject liable to suffer from the TSE.

11. The use of claim 10, wherein said level is measurable or detectable by mass spectroscopy.

12. The use of claim 10 or 11, wherein said haemoglobin, haemoglobin chain or truncated chain or fragment thereof has a molecular weight determinable by mass spectroscopy of about 15000 Da, 7500 Da or 3000 Da.

13. The use of any of claims 10 to 12, wherein the sample of body tissue is plasma.

14. The use of any of claims 10 to 13, wherein the sample of body tissue is from a living animal.

15. A bovine animal, or herd of said animals, that has or have been subjected to a test as defined in any of claims 10 to 14 and found to be free of a transmissible spongiform encephalopathy (TSE).
